(19) **Europäisches Patentamt**
**European Patent Office**
**Office européen des brevets**

(11) **EP 4 202 942 A1**

(12) **EUROPÄISCHE PATENTANMELDUNG**

(43) Veröffentlichungstag:
   **28.06.2023 Patentblatt 2023/26**

(21) Anmeldenummer: **21216717.5**

(22) Anmeldetag: **22.12.2021**

(51) Internationale Patentklassifikation (IPC):
   ***G16C 20/80*** *(2019.01)*   ***G16C 20/40*** *(2019.01)*

(52) Gemeinsame Patentklassifikation (CPC):
   **G16C 20/80; G16C 20/40;** G16C 20/70

(84) Benannte Vertragsstaaten:
   **AL AT BE BG CH CY CZ DE DK EE ES FI FR GB GR HR HU IE IS IT LI LT LU LV MC MK MT NL NO PL PT RO RS SE SI SK SM TR**
   Benannte Erstreckungsstaaten:
   **BA ME**
   Benannte Validierungsstaaten:
   **KH MA MD TN**

(71) Anmelder: **Bayer Aktiengesellschaft**
   **51373 Leverkusen (DE)**

(72) Erfinder: **Clevert, Djork-Arne**
   **10437, Berlin (DE)**

(74) Vertreter: **BIP Patents**
   **c/o Bayer Intellectual Property GmbH**
   **Alfred-Nobel-Straße 50**
   **40789 Monheim am Rhein (DE)**

(54) **BEREITSTELLEN VON INFORMATIONEN ZU CHEMISCHEN VERBINDUNGEN**

(57)   Die vorliegende Erfindung befasst sich mit der Bereitstellung von Informationen zu chemischen Verbindungen. Gegenstände der vorliegenden Erfindung sind ein Verfahren, ein Computersystem und ein Computerprogrammprodukt zur Bereitstellung von Informationen zu chemischen Verbindungen auf Basis von Strukturformeln der chemischen Verbindungen, die bildhaft auf einem Anzeigegerät dargestellt werden.

**Beschreibung**

Die vorliegende Erfindung befasst sich mit der Bereitstellung von Informationen zu chemischen Verbindungen. Gegenstände der vorliegenden Erfindung sind ein Verfahren, ein Computersystem und ein Computerprogrammprodukt zur Bereitstellung von Informationen zu chemischen Verbindungen auf Basis von Strukturformeln der chemischen Verbindungen, die bildhaft auf einem Anzeigegerät dargestellt werden.

Chemische Formeln sind heutzutage unverzichtbar bei der Beschreibung von chemischen Verbindungen und/oder chemischen Reaktionen.

Eine chemische Formel beschreibt die Zusammensetzung chemischer Verbindungen und kann Informationen über den Aufbau enthalten.

Die Summenformel gibt nur die stöchiometrische Zusammensetzung einer chemischen Verbindung wieder. Strukturformeln zeigen, wie die einzelnen Atome im Molekül miteinander verbunden sind (siehe z.B. J. K. Felixberger: Chemie für Einsteiger, Springer-Verlag 2017, ISBN: 978-3-662-52824-0, Kapitel 5.5 und S. Feil et al.: Faszinierende Chemie, Springer-Verlag 2017, ISBN: 978-3-662-49919-1, Seiten 32-33 und E. Benfenati et al.: Characterization of chemical structures, Chapter 3 of Quantitative Structure-Activity Relationships (QSAR) for Pesticide Regulatory Purposes, Elsevier-Verlag 2007, ISBN: 978-0-444-52710-3, Seiten 83 bis 109).

Chemische Verbindungen sind ferner über ihren IUPAC-Namen (IUPAC: *International Union of Pure and Applied Chemistry*) eindeutig identifizierbar.

In wissenschaftlichen Publikationen sowie in Patentanmeldungen und Patenten werden chemische Verbindungen oftmals anhand von Strukturformen bildhaft dargestellt.

Wissenschaftliche Publikationen sowie Patentanmeldungen und Patente sind heutzutage in digitaler Form verfügbar, d.h. sie lassen sich auf einem Anzeigegerät (z.B. einem Monitor) anzeigen.

Möchte man weitere Informationen zu einer in Form einer Strukturformel auf einem Anzeigegerät dargestellten chemischen Verbindung erhalten, muss man die chemische Struktur mit Hilfe eines Moleküleditors nachzeichnen, um sich beispielsweise mittels des Softwareprogramms CAS SciFinder® (CAS: *Chemical Abstract Service*) weitere Informationen zu der chemischen Verbindung anzuzeigen zu lassen. Alternativ kann man anhand des Namens der chemischen Verbindung nach Informationen in Datenbanken suchen. Oftmals ist der Name einer chemischen Verbindung (insbesondere der IUPAC-Name) aufgrund seiner Länge in einer grafischen Darstellung jedoch nicht angegeben, sondern die chemische Verbindung ist mit einer willkürlichen Ziffer versehen, anhand derer die chemische Verbindung außerhalb der Publikation nicht identifizierbar ist.

In jedem Fall sind Anstrengungen nötig, um weitere Informationen zu einer chemischen Verbindung, die mit Hilfe eines Softwareprogramms in Form ihrer Strukturformel bildhaft dargestellt wird, zu gewinnen.

Ausgehend vom beschriebenen Stand der Technik stellt sich damit die technische Aufgabe, die Bereitstellung von Informationen zu bildhaft anhand von Strukturformeln dargestellten chemischen Verbindungen zu erleichtern.

Dieses Problem wird durch die Gegenstände der unabhängigen Patentansprüche gelöst. Bevorzugte Ausführungsformen finden sich in den abhängigen Patentansprüchen sowie in der vorliegenden Beschreibung und in den Zeichnungen.

Ein erster Gegenstand der vorliegenden Erfindung ist ein computer-implementiertes Verfahren umfassend die Schritte:

- Empfangen einer Auswahl eines Benutzers, wobei die Auswahl eine auf einem Anzeigegerät angezeigte Strukturformel einer chemischen Verbindung betrifft,

- Erzeugen eines Eingabebildes der Strukturformel,

- Zuführen des Eingabebildes einem Modell des maschinellen Lernens, wobei das Modell des maschinellen Lernens konfiguriert und trainiert ist, eine eindeutige Kennung der chemischen Verbindung auf Basis des Eingabebildes bereitzustellen,

- Empfangen der eindeutigen Kennung der chemischen Verbindung von dem Modell des maschinellen Lernens,

- Abrufen von Informationen zu der chemischen Struktur auf Basis der eindeutigen Kennung aus einer oder mehreren Datenbanken,

- Anzeigen der Informationen auf dem Anzeigegerät.

Ein weiterer Gegenstand der vorliegenden Erfindung ist ein Computersystem umfassend

• eine Eingabeeinheit,

• eine Steuer- und Recheneinheit und

• ein Anzeigegerät,

- wobei die Steuer- und Recheneinheit konfiguriert ist, die Eingabeeinheit zu veranlassen, eine Auswahl von einem Benutzer zu empfangen, wobei die Auswahl eine auf dem Anzeigegerät angezeigte Strukturformel einer chemischen Verbindung betrifft,

- wobei die Steuer- und Recheneinheit konfiguriert ist, ein Eingabebild der Strukturformel zu erzeugen,

- wobei die Steuer- und Recheneinheit konfiguriert ist, das Eingabebild einem Modell des maschinellen Lernens zuzuführen, wobei das Modell des maschinellen Lernens konfiguriert und trainiert ist, eine eindeutige Kennung der chemischen Verbindung auf Basis des Eingabebildes bereitzustellen,

- wobei die Steuer- und Recheneinheit konfiguriert ist, die eindeutige Kennung der chemischen Verbindung von dem Modell des maschinellen Lernens zu empfangen,

- wobei die Steuer- und Recheneinheit konfiguriert ist, Informationen aus einer oder mehreren Datenbanken anhand der eindeutigen Kennung abzurufen,

- wobei die Steuer- und Recheneinheit konfiguriert ist, das Anzeigegerät zu veranlassen, die Informationen anzuzeigen.

Ein weiterer Gegenstand der vorliegenden Erfindung ist ein Computerprogrammprodukt umfassend ein Computerprogramm, das in einen Arbeitsspeicher eines Computersystems geladen werden kann und dort das Computersystem dazu veranlasst, folgende Schritte ausführen:

- Empfangen einer Auswahl eines Benutzers, wobei die Auswahl eine auf einem Anzeigegerät angezeigte Strukturformel einer chemischen Verbindung betrifft,

- Erzeugen eines Eingabebildes der Strukturformel,

- Zuführen des Eingabebildes einem Modell des maschinellen Lernens, wobei das Modell des maschinellen Lernens konfiguriert und trainiert ist, eine eindeutige Kennung der chemischen Verbindung auf Basis des Eingabebildes bereitzustellen,

- Empfangen der eindeutigen Kennung der chemischen Verbindung von dem Modell des maschinellen Lernens,

- Abrufen von Informationen zu der chemischen Struktur auf Basis der eindeutigen Kennung aus einer oder mehreren Datenbanken,

- Anzeigen der Informationen auf dem Anzeigegerät.

Die Erfindung wird nachstehend näher erläutert, ohne zwischen den Erfindungsgegenständen (Verfahren, Computersystem, Computerprogrammprodukt) zu unterscheiden. Die nachfolgenden Erläuterungen sollen vielmehr für alle Erfindungsgegenstände in analoger Weise gelten, unabhängig davon, in welchem Kontext (Verfahren, Computersystem, Computerprogrammprodukt) sie erfolgen.

Mit Hilfe der vorliegenden Erfindungen können Informationen zu chemischen Verbindungen, die in Form einer Strukturformel auf einem Anzeigegerät angezeigt werden, einfach und effizient aus einer oder mehreren Datenbanken abgerufen und angezeigt werden. Dabei reichen beispielsweise ein Mausklick oder wenige Mausklicke, um die Informationen anzuzeigen.

Unter dem Begriff "chemische Verbindung" wird ein Reinstoff verstanden, der aus Atomen von zwei oder mehreren chemischen Elementen besteht, wobei (im Gegensatz zu Gemischen) die Atomarten zueinander in einem festen Verhältnis stehen. Das zahlenmäßige Verhältnis der Atome zueinander wird durch chemische Bindungen zwischen den Atomen bestimmt, und das Verhältnis lässt sich in einer Summenformel darstellen. Eine Summenformel ist jedoch oft nicht eindeutig, da isomere Verbindungen dieselbe Summenformel haben, aber unterschiedliche Verbindungen sind. Charakteristisch für jede chemische Verbindung ist ihre eindeutige chemische Struktur.

Vorzugsweise handelt es sich bei der chemischen Verbindung um eine organische Verbindung. Eine organische Verbindung ist eine chemische Verbindung, die Kohlenstoff-Wasserstoff-Bindungen (C-H-Bindungen) umfasst. Vorzugsweise handelt es sich um eine organische Verbindung, deren Moleküle ausschließlich die folgenden Elemente umfassen: Kohlenstoff (C), Wasserstoff (H), Sauerstoff (O), Stickstoff (N), Schwefel (S), Fluor (F), Chlor (Cl), Brom (Br), Iod (I) und/oder Phosphor (P).

Unter dem Begriff "Strukturformel" wird die Darstellung einer chemischen Verbindung bezeichnet, aus der hervorgeht, wie die einzelnen Atome miteinander verbunden sind. Beispiele für chemische Strukturformeln sind Elektronenformel, Valenzstrichformel, Keilstrichformel, Skelettformel und Konstitutionsformel. Vorzugsweise wird die chemische Verbindung in Form der Valenzstrichformel, Keilstrichformel, Skelettformel, Konstitutionsformel oder einer Mischung der genannten Formeln angezeigt. So kann es beispielsweise sein, dass eine oder mehrere Atomgruppen in einer ansonsten als Valenzstrichformel dargestellten Repräsentation durch Konstitutionsformeln dargestellt werden. Ein typisches Beispiel ist eine organische Säuregruppe, die oftmals in Form der Konstitutionsformel -COOH dargestellt wird, oder eine Aldehydgruppe, die oftmals in Form der Konstitutionsformel -CHO dargestellt wird, oder eine Alkoholgruppe, die oftmals in Form der Konstitutionsformel -COH dargestellt wird.

Die chemische Verbindung wird anhand einer Strukturformel mittels eines Softwareprogramms auf einem Anzeigegerät angezeigt, d.h. bildhaft dargestellt. Bei dem Softwareprogramm kann es sich beispielsweise um eine Software zum Anzeigen von Dokumenten des Typs PDF handeln (PDF: Portable Document Format). Als Beispiel sei das von der Firma Adobe Inc. angebotene Softwareprogramm Adope Acrobat® Reader genannt. Es kann sich bei dem Softwareprogramm aber auch um einen Internetbrowser handeln, der grafische Darstellungen von Strukturformeln anzeigen kann. Eine Vielzahl von weiteren Möglichkeiten, Strukturformeln auf einem Anzeigegerät anzuzeigen, sind dem Fachmann bekannt.

Bei dem Anzeigegerät handelt es sich üblicherweise um einen Monitor. Beispiele für Monitore sind u.a. zu finden in: https://19january2017snapshot.epa.gov/sites/production/files/2014-01/documents/computer_display_profile.pdf.

In einem ersten Schritt wird eine Auswahl eines Benutzers empfangen. Die Auswahl betrifft die auf dem Anzeigegerät angezeigte Strukturformel. Werden mehrere Strukturformeln angezeigt, betrifft die Auswahl eine der angezeigten Strukturformeln.

Durch die Auswahl spezifiziert der Benutzer also diejenige Strukturformel, zu der er nähere Informationen in Erfahrung bringen möchte.

Die Auswahl kann beispielsweise mit einer Computermaus (kurz: Maus), einem Eingabestift und/oder einem Finger auf einem berührungsempfindlichen Anzeigegerät, einem Trackball, einem Joystick, mittels Gestensteuerung und/oder dergleichen erfolgen.

Der Benutzer kann zur Auswahl beispielsweise einen rechteckigen Rahmen um die jeweilige Strukturformel ziehen. Die geschieht üblicherweise, in dem der Benutzer zunächst eine erste Ecke des Rahmens z.B. durch einen Mausklick definiert und dann durch diagonales Ziehen der Maus über die Strukturformel diejenige Ecke definiert, die in dem Rahmen den größten Abstand zur ersten Ecke aufweist. Die übrigen Ecken ergeben sich automatisch, wenn angenommen wird, dass das Rechteck horizontal und vertikal entlang der Rasteranordnung der Monitor-Bildpunkte ausgerichtet ist.

Der Benutzer kann die Strukturformel auch durch Einzeichnen einer geschlossenen Kurve um die Strukturformel spezifizieren. Eine solche Möglichkeit ist aus Bildbearbeitungsprogrammen unter dem Begriff "Lasso-Werkzeug" bekannt.

Der Benutzer kann die Strukturformel z.B. durch Anklicken eines Atomsymbols oder einer chemischen Bindung, das/die Bestandteil der Strukturformel ist, mit einer Maus auswählen. Das erfindungsgemäße Computerprogramm kann konfiguriert sein, alle Komponenten, die zu einer Strukturformel gehören, automatisch zu erkennen, wenn nur eine Komponente (z.B. ein Atomsymbol oder eine chemische Bindung) spezifiziert ist. Üblicherweise erstrecken sich chemische Bindungen in Form eines Graphen über die gesamte Strukturformel. Alle Pixel des Graphen sind Bestandteil der Strukturformel. Elementsymbole oder Atomgruppen wie beispielsweise OH-Gruppen lassen sich über ihre Nähe zu den Bindungen und/oder anderen Elementsymbolen der Strukturformel als zu der Strukturformel zugehörig definieren: alle Elementsymbole, Ladungssymbole, freie Elektronenpaare, Atomgruppen und/oder sonstigen möglichen (üblichen) Bestandteile einer Summenformel, bei denen der kürzeste Abstand zu einem Bestandteil der Strukturformel eine vordefinierte maximale Distanz nicht überschreitet, zählen als zu der Strukturformel zugehörig; mögliche Bestandteile mit einem größeren Abstand werden nicht zu der Strukturformel gezählt.

Es ist auch möglich, dass eine Strukturformel (allein) dadurch durch einen Benutzer ausgewählt wird, indem der Benutzer mit einen Mauszeiger auf dem Anzeigegerät über die Strukturformel fährt. Das erfindungsgemäße Computerprogramm kann konfiguriert sein, die Bewegung des Mauszeigers innerhalb einer grafischen Benutzeroberfläche auf dem Anzeigegerät zu verfolgen. Innerhalb der grafischen Benutzeroberfläche befindet sich mindestens eine Strukturformel. Das Computerprogramm kann konfiguriert sein, für jedes Pixel, den die Spitze des Mauszeigers (oder ein anderer Bestandteil des Mauszeigers) überfährt (oder berührt), zu prüfen, ob es ein Bestandteil einer Strukturformel ist. Dazu kann zunächst der Farbwert des überfahrenen (berührten) Pixels ermittelt werden. Üblicherweise sind Strukturformeln in schwarz (oder grau) auf einem hellen (meist weißen) Hintergrund abgebildet. Alle Pixel, die keinen üblichen Farbton einer Strukturformel aufweisen (z.B. schwarz oder grau), können vernachlässigt werden. Für alle Pixel, die einen Farbton aufweisen, der üblicherweise bei einer Strukturformel verwendet wird (z.B. schwarz oder grau) können diejenigen Pixel ermittelt werden, die mit dem überfahrenen (berührten) Pixel zusammenhängen. Dabei bedeutet der Begriff "zusammenhängen", dass man von dem überfahrenen (berührten) Pixel zu allen zusammenhängenden Pixeln gelangt, indem man sich lediglich entlang einer Route von zusammenhängenden Pixeln bewegt. Ist ein Graph zusammenhängender Pixel identifiziert, kann geprüft werden, ob es sich bei den zusammenhängenden Pixeln um eine Strukturformel (oder einen Teil einer Strukturformel) handelt. Diese Prüfung kann beispielsweise mit Hilfe eines Modells des maschinellen Lernens erfolgen. Ein solches Modell des maschinellen Lernens kann konfiguriert und trainiert sein, eine Gruppe zusammenhängender Pixel in eine von zwei Klassen einzuordnen: eine erste Klasse, die Strukturformeln umfasst und eine zweite Klasse, die keine Strukturformeln umfasst. Mit anderen Worten, das Modell des maschinellen Lernens kann konfiguriert und trainiert sein, eine Gruppe von Pixeln, die Bestandteil einer Strukturformel ist, von Gruppen von Pixeln, die zu keiner Strukturformel gehören, zu unterscheiden.

Ist von einem Benutzer eine Auswahl getroffen worden, die eine Strukturformel einer chemischen Verbindung betrifft, wird ein Eingabebild von der Strukturformel erzeugt.

Ein solches Eingabebild ist eine Repräsentation der Strukturformel, die einem Modell des maschinellen Lernens zugeführt werden kann.

Viele Modelle des maschinellen Lernens benötigen zur Verarbeitung Eingabedaten definierter Größe (Dimension) in Form von Zahlen.

Dementsprechend kann es sich bei dem Eingabebild um eine digitale Rastergrafik handeln. Rastergrafiken umfassen eine rasterförmige Anordnung von so genannten Bildpunkten (Pixel), denen jeweils ein Farbton bzw. ein Grauton zugeordnet ist. Die Hauptmerkmale einer Rastergrafik sind daher die Bildgröße (Breite und Höhe gemessen in Pixel, umgangssprachlich auch Bildauflösung genannt) sowie die Farbtiefe. Einem Bildpunkt einer digitalen Bilddatei ist üblicherweise eine Farbe (ein Farbton) zugeordnet. Die für einen Bildpunkt verwendete Kodierung der Farbe definiert sich unter anderem über den Farbraum und die Farbtiefe. Der einfachste Fall ist ein Binärbild, bei dem ein Bildpunkt einen Schwarzweiß-Wert speichert. Bei einem Bild, dessen Farbe über den so genannten RGB-Farbraum definiert ist (RGB steht für die Grundfarben Rot, Grün und Blau), verfügt jeder Bildpunkt über drei Kanäle, einem Kanal für die Farbe Rot, einem Kanal für die Farbe Grün und einem Kanal für die Farbe Blau. Die Farbe eines Bildpunktes ergibt sich durch die Überlagerung (additives Mischen) der Farbwerte der Subpixel. Der Farbwert eines Kanals kann beispielsweise in 256 Farbnuancen unterteilt sein, die Tonwerte genannt werden und üblicherweise von 0 bis 255 reichen. Die Farbnuance "0" eines jeden Farbkanals ist die dunkelste. Haben alle drei Kanäle den Tonwert 0, erscheint der entsprechende Bildpunkt schwarz; haben alle drei Kanäle den Tonwert 255, erscheint der entsprechende Bildpunkt weiß.

Der Begriff "digital" bedeutet, dass die Rastergrafik von einer Maschine, in der Regel einem Computersystem, verarbeitet werden kann. Unter "Verarbeitung" werden die bekannten Verfahren zur elektronischen Datenverarbeitung (EDV) verstanden.

Vorzugsweise handelt es sich bei dem Eingabebild um eine binäre Rastergrafik mit einer definierten Bildgröße (z.B. 512 x 512 Pixel).

Je nach Verfahren, wie die Strukturformel durch einen Benutzer ausgewählt worden ist, gibt es verschiedene Verfahren der Erzeugung eines Eingabebildes.

Hat der Benutzer die Strukturformel durch Aufspannen eines rechteckigen Rahmens ausgewählt, kann der Bereich innerhalb des Rahmens zur Erzeugung des Eingabebildes dienen.

Das erfindungsgemäße Computerprogramm kann konfiguriert sein, auf den Bereich des Arbeitsspeichers des entsprechenden Computersystems zuzugreifen, der für die Anzeige von Informationen (insbesondere der Strukturformel) verantwortlich ist. Ein vom Benutzer ausgewählter rechteckiger Bereich kann als digitale Rastergrafik direkt in ein Modell des maschinellen Lernens eingegeben werden. Verlangt das Modell des maschinellen Lernens nach einer Eingabedatei definierter Größe, kann für den Fall, dass der ausgewählte Bereich kleiner ist als die verlangte Größe, ein Rahmen um den Bereich gelegt werden, der den Bereich ergänzt, oder der Bereich mit einem Upsampling-Verfahren auf die geforderte Größe skaliert werden. Für den Fall, dass der ausgewählte Bereich größer ist als die geforderte Größe, kann ein Downsampling-Verfahren verwendet werden, um den Bereich auf die geforderte Größe zu skalieren. Es ist auch denkbar, Bereiche des Rechtecks, die keine Pixel umfassen, die zu der Strukturformel gehören, wegzuschneiden. Verfahren zum Skalieren von Bildern (Up-/Downsampling) sind im Stand der Technik beschrieben (siehe z.B.: T. Frajka et al.: Downsampling dependent upsampling of images, Signal Processing: Image Communication, Vol. 19, Issue 3, 2004, pages 257-265; US8121434B2; M.-S. Lee et al.: An Efficient Upsampling Technique for Images and Videos, in: P. Muneesawang et al. (eds): Advances in Multimedia Information Processing - PCM 2009, Lecture Notes in Computer Science, Vol. 5879, Springer).

Hat der Benutzer eine geschlossene Kurve um die Strukturformel gezogen, kann der entsprechende Bereich innerhalb der Kurve (d.h. die in dem Bereich liegenden Pixel) erfasst und auf einem neutralen Hintergrund (z.B. ein Rechteck bestehend aus neutralen Pixeln) mit der geforderten Größe platziert werden. Ist der Bereich zu groß, kann er auf einen größeren Hintergrund platziert werden und die resultierende Bilddatei durch Downsampling in ihrer Bildgröße verkleinert werden.

Hat der Benutzer die Strukturformel durch Anklicken eines Bestandteiles mit einem Mauszeiger oder durch Überfahren oder Berühren eines Bestandteils mit einem Mauszeiger ausgewählt, kann die identifizierte Gruppe zusammenhängender Pixel auf einem neutralen Hintergrund (z.B. ein Rechteck bestehend aus weißen Pixeln) platziert werden. Ist der Bereich zu groß, kann er auf einen größeren Hintergrund platziert werden und die resultierende Bilddatei durch Downsampling in ihrer Bildgrößer verkleinert werden.

Das Eingabebild wird in einem nächsten Schritt einem Modell des maschinellen Lernens zugeführt. Das Modell des maschinellen Lernens ist konfiguriert und trainiert, auf Basis des Eingabebildes eine eindeutige Kennung für die chemische Verbindung, deren Strukturformel durch das Eingabebild repräsentiert wird, zu bestimmen.

Der Begriff "Modell des maschinellen Lernens", wie er in dieser Beschreibung verwendet wird, kann als eine computerimplementierte Datenverarbeitungsarchitektur verstanden werden. Das Modell des maschinellen Lernens kann Eingangsdaten empfangen und Ausgangsdaten auf der Grundlage dieser Eingangsdaten und Parametern des Modells des maschinellen Lernens bereitstellen. Das Modell des maschinellen Lernens kann durch Training eine Beziehung zwischen Eingabedaten und Ausgabedaten erlernen. Beim Training können die Parameter des Modells angepasst werden, um eine gewünschte Ausgabe für eine bestimmte Eingabe zu liefern.

Die Ausgabe des Modells des maschinellen Lernens kann ein Klassifizierungsergebnis, eine Datenkompression, ein Rekonstruktionsergebnis, ein Regressionsergebnis und/oder ein anderes Ergebnis sein, wie in dieser Beschreibung beschrieben.

Beim Training eines Modells des maschinellen Lernens werden dem Modell Trainingsdaten zum Lernen zur Verfügung gestellt. Der Begriff "trainiertes Modell des maschinellen Lernens" bezieht sich auf das Modell, das durch den Trainingsprozess erstellt wird. Die Trainingsdaten müssen die richtige Antwort enthalten, die als Zieldaten (engl.: *traget*) bezeichnet werden. Der Lernalgorithmus findet in den Trainingsdaten Muster, die die Eingabedaten auf die Zieldaten abbilden, und gibt ein trainiertes Modell aus, das diese Muster erfasst.

Im Trainingsprozess werden Trainingsdaten in das Modell des maschinellen Lernens eingegeben und das Modell erzeugt eine Ausgabe. Die Ausgabe wird mit den (bekannten) Zieldaten verglichen. Die Parameter des Modells werden so verändert, dass die Abweichungen zwischen der Ausgabe und den (bekannten) Zieldaten auf ein (definiertes) Minimum reduziert werden.

Im Allgemeinen kann eine Verlustfunktion (engl. *loss function*) für das Training verwendet werden, um den Trainingsprozess zu steuern. Eine Verlustfunktion kann zum Beispiel eine Vergleichsmetrik zwischen der Ausgabe und den Zieldaten beinhalten. Die Verlustfunktion kann so gewählt werden, dass sie eine erwünschte Beziehung zwischen Ausgabe und Zieldaten belohnt und/oder eine unerwünschte Beziehung zwischen einer Ausgabe und Zieldaten bestraft. Eine solche Beziehung kann z.B. eine Ähnlichkeit, eine Unähnlichkeit oder eine andere Beziehung sein.

Eine Verlustfunktion kann verwendet werden, um einen Verlustwert (engl. *loss value*) für ein gegebenes Paar aus Ausgabe und Zieldaten zu berechnen. Das Ziel des Trainingsprozesses kann darin bestehen, die Parameter des Modells des maschinellen Lernens so zu verändern (anzupassen), dass der Verlustwert auf ein (definiertes) Minimum reduziert wird.

Eine Verlustfunktion kann z.B. die Abweichung zwischen der Ausgabe des Modells für bestimmte Eingabedaten und den Zieldaten quantifizieren. Wenn beispielsweise die Ausgabe und die Zieldaten Zahlen sind, kann die Verlustfunktion die absolute Differenz zwischen diesen Zahlen sein. In diesem Fall kann ein hoher absoluter Wert der Verlustfunktion bedeuten, dass ein Parameter des Modells signifikant verändert werden muss.

Bei vektoriellen Ausgaben können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder jede andere Art von Differenzmetrik zweier Vektoren gewählt werden.

Bei höherdimensionalen Ausgaben, wie z.B. zweidimensionalen, dreidimensionalen oder höherdimensionalen Ausgaben, kann z. B. eine elementweise Differenzmetrik verwendet werden. Alternativ oder zusätzlich können die Ausgabedaten vor der Berechnung eines Verlustwertes transformiert werden, z.B. in einen eindimensionalen Vektor.

Wie beschrieben ist das Modell des maschinellen Lernens der vorliegenden Erfindung konfiguriert und trainiert, auf Basis eines Eingabebildes eine eindeutige Kennung für die chemische Verbindung, deren Strukturformel durch das Eingabebild repräsentiert wird, zu bestimmen.

Das Modell kann beispielsweise in einem überwachten Lernverfahren anhand einer Vielzahl von Eingabebildern von Strukturformeln chemischer Strukturen und eindeutigen Kennungen zu den chemischen Strukturen trainiert werden, die Eingabebilder auf die chemischen Strukturen abzubilden. Der Begriff "Vielzahl" bedeutet eine Anzahl von vorzugsweise mehr als einhundert, noch mehr bevorzugt von mehr als eintausend.

Unter dem Begriff "eindeutige Kennung" wird vorzugsweise ein alphanumerischer Code verstanden, anhand dessen eine chemische Verbindung eindeutig identifiziert werden kann. "Eindeutig" bedeutet, dass es für jede chemische Verbindung genau eine Kennung gibt und für jede Kennung genau eine Verbindung. In der Mathematik wird für eine solche Relation auch der Begriff "eineindeutig" verwendet.

Bei der eindeutigen Kennung kann es sich beispielsweise um den IUPAC-Namen, den SMILES-Code, den InChI-Code, den CML-Code, den WLN-Code oder eine vergleichbare Kennung handeln.

SMILES steht für *Simplified Molecular Input Line Entry Specification*. Dabei handelt es sich um einen chemischen Strukturcode, bei dem die Struktur beliebiger Moleküle stark vereinfacht als ASCII-Zeichenkette wiedergegeben wird. *Chemical Markup Language* (ChemML oder CML) ist ein auf XML (*Extensible Markup Language*) basierendes Datenformat, das unter anderem zur Darstellung von chemischen Formeln verwendet werden kann. Die *Wiswesser Line Notation* (WLN) und der *IUPAC International Chemical Identifier* (InChI) sind weitere Beispiele von Strukturcodes, die es ermöglichen, die Struktur eines Moleküls eindeutig in eine standardisierte Zeichenkette zu übersetzen.

SMILES-Code, InChI-Code, CML-Code, WLN-Code und vergleichbare Codes werden in dieser Beschreibung auch als Strukturcode bezeichnet. Sie geben die chemische Struktur in Form einer Zeichenkette wieder. Eine solche Zeichenkette umfasst Zeichen eines definierten Zeichensatzes, d.h. eines definierten Vorrats an Elementen. Solche Elemente (Zeichen) können unter anderem die Buchstaben eines Alphabetes, Ziffern, aber auch andere Symbole sein, etwa Sonderzeichen, Satzzeichen, die Zeichen der Lautumschrift des IPA-Codes (IPA: Internationales Phonetisches Alphabet) oder der Brailleschrift, Piktogramme verschiedener Art oder Steuerzeichen. Beispiele für bekannte Zeichensätze sind ASCII (*American Standard Code for Information Interchange*), EBCDIC (*Extended Binary Coded Decimal Interchange Code*) und Unicode.

Vorzugsweise wird als eindeutige Kennung ein Strukturcode verwendet, bei dem die Abbildung einer Strukturformel einer chemischen Verbindung auf den Strukturcode eindeutig (für jede chemische Struktur gibt es genau einen Strukturcode) und reversibel ist (aus dem Strukturcode lässt sich die Strukturformel rekonstruieren, z.B. in Form einer bildhaften Darstellung).

In einer bevorzugten Ausführungsform handelt es sich bei der eindeutigen Kennung um einen kanonischen SMILES-Code. In der Regel können für ein Molekül mehrere gleichermaßen gültige SMILES-Code erzeugt werden. Daher wurden Algorithmen entwickelt, um dieselbe SMILES-Zeichenkette für ein bestimmtes Molekül zu erzeugen; von den vielen möglichen Zeichenketten wählen diese Algorithmen nur eine aus (siehe z.B. D. Weininger et al.: SMILES. 2. algorithm for generation of unique SMILES notation, J Chem Inf Comp Sci 1989, 29(2):97e101). Kanonische SMILES-Codes sind für jede Struktur eindeutig.

Die genannten Strukturcodes haben die Gemeinsamkeit, dass die Größe des Strukturcodes (z.B. die Länge der Zeichenkette) variabel ist und von der Komplexität und/oder Größe des Moleküls abhängig ist.

In einer bevorzugten Ausführungsform wird das Modell des maschinellen Lernens nicht trainiert, eine (direkte) Abbildung eines Eingabebildes auf einen Strukturcode variabler Größe wie beispielsweise einen SMILES-Code vorzunehmen, sondern auf eine Repräsentation der Struktur definierter (fester) Größe.

Ein solches Training kann beispielsweise mit Hilfe von zwei künstlichen neuronalen Netzwerken erfolgen.

Zunächst wird ein erstes Netzwerk trainiert, Strukturcodes variabler Größe in komprimierte Repräsentationen der Struktur definierter (fester) Größe zu verwandeln. Dazu kann ein Autoencoder verwendet werden. Der Encoder des Autoencoders erzeugt auf Basis eines Strukturcodes variabler Größe eine komprimierte Repräsentation definierter (fester) Größe. Der Decoder rekonstruiert den Strukturcode variabler Größe aus der komprimierten Repräsentation fester Größe. Der Vorteil eines solchen Autoencoders liegt unter anderem darin, dass er in einem unüberwachten Lernverfahren (und damit automatisierbar) auf Basis einer Vielzahl an Strukturcodes trainiert werden kann.

Vorzugsweise basiert der Autoencoder auf dem Autoencoder von Winter et al. (Chem. Sci., 2019, 10, 1692-1701). Dieser Autoencoder kann beispielsweise trainiert werden, einen SMILES-Code einer chemischen Verbindung in den entsprechenden kanonischen SMILES-Code zu übersetzen. Die Architektur des Autoencoders weist einen Engpass auf. Eine SMILES-Zeichenfolge wird durch den Encoder in einen 512-dimensionalen Merkmalsvektor kodiert. Dieser Merkmalsvektor ist ein Beispiel für eine komprimierte Repräsentation definierter (fester) Größe. Der Decoder erzeugt aus der komprimierten Repräsentation definierter (fester) Größe dann den kanonischen SMILES-Code.

Der Autoencoder wird vorzugsweise in einem unüberwachten Lernverfahren (*unsupervised learning*) trainiert, den Rekonstruktionsfehler auf Einzelzeichenebene für jede Eingabesequenz zu minimieren. Das Trainieren kann automatisiert erfolgen; d.h. der Autoencoder kann automatisiert anhand von Strukturcodes von chemischen Strukturen einer Vielzahl von chemischen Referenzverbindungen lernen, diese Strukturcodes variabler Größe in komprimierte Repräsentationen fester Größe zu transformieren und aus den komprimierten Repräsentationen die Strukturcodes variabler Größe zu rekonstruieren. Strukturcodes von chemischen Strukturen einer Vielzahl von chemischen Referenzverbindungen können Datenbanken wie beispielsweise PubChem (https://pubchem.ncbi.nlm.nih.gov/) entnommen werden. Solchen Datenbanken können auch bildhafte Darstellungen der chemischen Strukturen einer Vielzahl von chemischen Referenzverbindungen entnommen werden.

Weitere Beispiele und Details zu Autoencodern und deren Erzeugung sind beispielsweise zu finden in Q. V. Le: A Tutorial on Deep Learning Part 2: Autoencoders, Convolutional Neural Networks and Recurrent Neural Networks, 2015, https://cs.stanford.edu/~quocle/tutorial2.pdf; W. Meng: Relational Autoencoder for Feature Extraction, arXiv: 1802.03145vl; WO2018046412A1.

Ist der Autoencoder trainiert, können für jede chemische Verbindung einer Vielzahl von chemischen Verbindungen Paare von Strukturformel der chemischen Verbindung und der komprimierten Repräsentation definierter (fester) Größe erzeugt werden. Die komprimierten Repräsentationen definierter Größe lassen sich mit Hilfe des Encoders eines Autoencoders auf Basis der Strukturcodes variabler Länge (z.B. den SMILES-Codes) erzeugen. Diese Paare können zum Trainieren des zweiten künstlichen neuronalen Netzwerks verwendet werden.

Das zweite künstliche neuronale Netzwerk wird trainiert, aus einem Eingabebild einer Strukturformel einer chemischen Verbindung die komprimierte Repräsentation der Struktur der chemischen Verbindung zu generieren. Wenn $\mathbb{F} \subset \mathbb{R}^{k \times l}$ der Bildraum mit der Dimension $k \times l$ ist, (mit $k$ und $l$ der Anzahl an Pixeln in Richtung der beiden Dimensionen des Eingangsbildes) und $\mathbb{C} \subset [-1, 1]^c$ der Raum der komprimierten Repräsentation mit der Dimension c (z.B. 512), dann ist das zweite künstliche neuronale Netzwerk vorzugsweise ein Regressionsmodell, das eine Abbildung von dem Bildraum auf den Raum der komprimierten Repräsentation vornimmt; d.h. $f_\theta : \mathbb{F} \rightarrow \mathbb{C}$, wobei $\theta$ ein Satz von trainierbaren Modellparametern ist. Das Trainieren kann durch Minimieren der Distanz $l(d) = (l(\text{csc}_{\text{true}} - \text{csc}_{\text{predicted}})$ erfolgen, wobei $l$ beispielsweise die L2-regularisierte Fehlerfunktion sein kann und $\text{csc}_{\text{true}}$ der korrekte komprimierten Strukturcode und $\text{csc}_{\text{predicted}}$ der vorhergesagte komprimierte Strukturcode sein kann. Details zu Fehlerfunktionen können der Literatur entnommen werden (siehe. z.B. F. Nie et al.: An investigation for loss functions widely used in machine learning; Communications in Information and Systems, 2018, 18. 37-52. 10.4310/CIS.2018.vl8.nl.a2).

Vorzugsweise wird die von Clevert et al. beschriebene Architektur für das zweite neuronale Netzwerk verwendet (Img2Mol - accurate SMILES recognition from molecular graphical depictions, Chem. Sci., 2021,12, 14174-14181, siehe auch die "Supplementary Files" unter https://doi.org/10.1039/D1SC01839F).

Sind die beiden künstlichen neuronalen Netzwerke trainiert, können sie zu einem Modell des maschinellen Lernens gemäß der vorliegenden Erfindung verknüpft werden.

Die Ausgabeschicht des zweiten künstlichen neuronalen Netzwerks, die eine komprimierte Repräsentation definierter Größe auf Basis eines Eingabebildes erzeugt, kann mit dem Eingang des Decoders des trainierten Autoencoders verbunden werden (der Encoder kann verworfen werden). Der Decoder empfängt die komprimierte Repräsentation der Struktur einer chemischen Verbindung und wandelt sie in einen Strukturcode variabler Größe (zum Beispiel einen kanonischen SMILES-Code) um. Ein solcher Strukturcode variabler Größe kann zur Identifizierung von Informationen zu der chemischen Verbindung in einer oder mehreren Datenbanken verwendet werden. Natürlich ist es grundsätzlich denkbar, die komprimierte Repräsentation definierter Größe (z.B. in Form eines Vektors) als eindeutige Kennung der chemischen Verbindung zu verwenden und Informationen über die chemische Verbindung unter dieser Kennung abzurufen. Allerdings ist die hier beschriebene komprimierte Repräsentation keine so gängige Größe wie beispielsweise ein kanonischer SMILES-Code, so dass die hier beschriebene Transformation der komprimierten Repräsentation in einen Strukturcode variabler Größe letztlich dazu dient, eine gängige (d.h. weit verbreitete) Repräsentation der chemischen Verbindung zu erzeugen, unter der Informationen aus einer Vielzahl an Datenbanken abgerufen werden können.

In einem weiteren Schritt werden dann auf Basis der eindeutigen Kennung Informationen zu der chemischen Verbindung aus einer oder mehreren Datenbanken abgerufen und auf dem Anzeigegerät angezeigt.

Bei den Informationen kann es sich beispielsweise um die folgenden Merkmale (Eigenschaften) zu der chemischen Verbindung handeln: Name (z.B. IUPAC-Name oder ein anderer Name), Summenformel, CAS-Nummer, molare Masse, Aggregatzustand unter Standardbedingungen, Dichte unter Standardbedingungen, Schmelzpunkt, Siedepunkt, Löslichkeit in Wasser oder einem anderen Lösemittel, Sicherheitshinweise zum Umgang mit der Verbindung, toxikologische Daten, Wirkdaten (bei Wirkstoffen) und/oder andere/weitere.

Es kann auch die Strukturformel der chemischen Verbindung angezeigt werden (z.B. neben der bereits angezeigten Strukturformel, auf Basis der das Modell des maschinellen Lernens eine eindeutige Kennung identifiziert hat), damit der Benutzer prüfen kann, ob die chemische Verbindung korrekt identifiziert worden ist. So kann das Ergebnis der Erzeugung einer eindeutigen Kennung auf Basis einer angezeigten Strukturformel unmittelbar überprüft und bewertet werden, indem die beiden angezeigten Strukturen miteinander verglichen werden. Bei einem korrekten Ergebnis stimmen die beiden angezeigten Strukturen überein. Bei einem fehlerhaften Ergebnis kann durch Identifikation der Abweichungen in den Strukturen ermittelt werden, mit welchen Strukturmerkmalen das Modell des maschinellen Lernens noch Probleme haben. Anhand der Abweichungen können Strukturen für ein weiteres (aufbauendes) Training ermittelt und/oder ausgewählt und/oder erzeugt werden. Auch ein automatisiertes verstärkendes Lernen (engl.: reinforced learning) ist denkbar.

Die vorliegende Erfindung kann ganz oder teilweise mit einem Computersystem ausgeführt werden. Ein "Computersystem" ist ein System zur elektronischen Datenverarbeitung, das mittels programmierbarer Rechenvorschriften Daten verarbeitet. Ein solches System umfasst üblicherweise eine Steuer- und Recheneinheit, oftmals auch als "Computer" bezeichnet, diejenige Einheit, die einen Prozessor zur Durchführung logischer Operationen und einen Arbeitsspeicher zum Laden eines Computerprogramms umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabemittel dienen. Beispiele hierfür sind Monitor (Bildschirm), Drucker, Scanner, Maus, Tastatur, Joystick, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Eingaben in das Computersystem (zum Bespiel zur Steuerung durch einen Nutzer) erfolgen über Eingabemittel wie beispielsweise eine Tastatur, eine Maus, ein Mikrofon, ein berührungsempfindliches Display und/oder dergleichen. Ausgaben erfolgen über Ausgabemittel, die insbesondere ein Monitor (Bildschirm), ein Drucker und/oder ein Datenspeicher sein können.

Die Erfindung wird nachstehend anhand von Zeichnungen näher erläutert, ohne die Erfindung auf die in den Zeichnungen gezeigten Merkmale oder Merkmalkombinationen beschränken zu wollen.

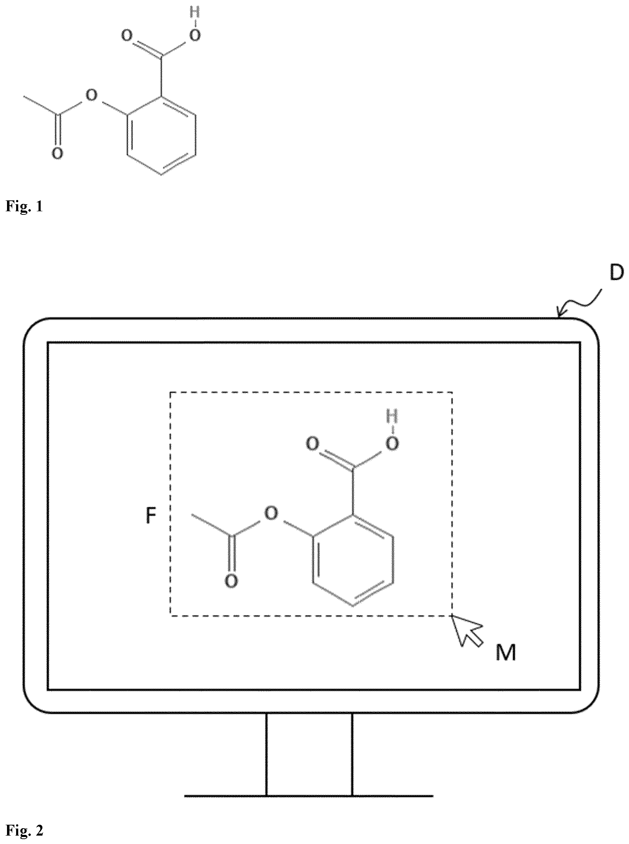

Fig. 1 zeigt ein Beispiel für eine bildhafte Darstellung der Strukturformel einer chemischen Verbindung. Bei der Verbindung handelt es sich um Acetylsalicylsäure; der IUPAC-Name der Verbindung lautet 2-(Acetyloxy)benzoesäure (engl.: 2-acetoxybenzoic acid). Bei der bildhaft dargestellten Strukturformel handelt es sich um eine Skelettformel: die Kohlenstoffatome und die an Kohlenstoffatome gebundenen Wasserstoffatome sind nicht explizit dargestellt; lediglich die Sauerstoffatome (O) und das mit einem Sauerstoffatom verbundene Wasserstoffatom (H) sind durch ihre Elementsymbole (O, H) explizit dargestellt. Es ist denkbar, dass Heteroatome (wie beispielsweise die Sauerstoffatome in der in Fig. 1 gezeigten Skelettformel) farblich hervorgehoben sind.

Der InChI-Code für die in Fig. 1 gezeigte chemische Struktur der chemischen Verbindung 2-(Acetyloxy)benzoesäure lautet: 1S/C9H8O4/c1-6(10)13-8-5-3-2-4-7(8)9(11)12/h2-5H,1H3,(H,11,12). Der SMILES-Code für die in Fig. 1 gezeigte chemische Struktur der chemischen Verbindung 2-(Acetyloxy)benzoesäure lautet: CC(=O)OC1=CC=CC=C1C(=O)O.

Fig. 2 zeigt schematisch und beispielhaft, wie eine Strukturformel auf einem Anzeigegerät (D) angezeigt wird. Ein Benutzer hat mit einem Mauszeiger (M) einen Rahmen (F) um die Strukturformel gezogen, um die Strukturformel auszuwählen. Der Bereich innerhalb des Rahmens (F) kann (ggf. nach Skalierung und/oder Wegschneiden von Bereichen, die keine Strukturformel umfassen und/oder Ergänzen eines Rahmens) als Eingabebild verwendet werden.

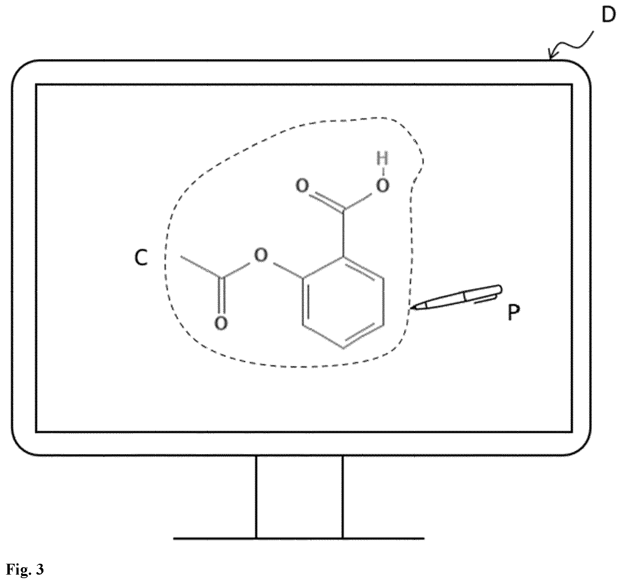

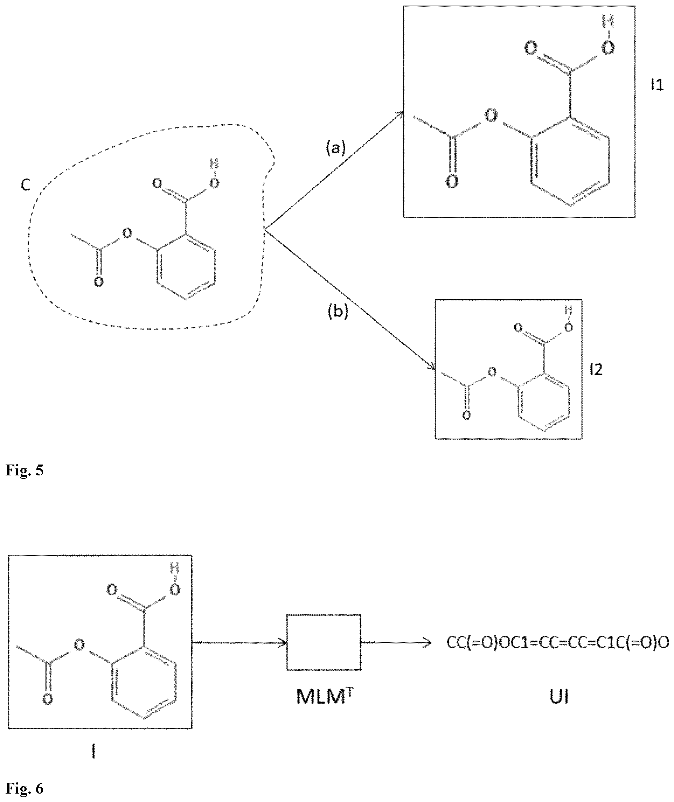

Fig. 3 zeigt beispielhaft und schematisch eine weitere Möglichkeit, wie ein Benutzer eine Strukturformel auswählen kann. Die Strukturformel wird, wie im Fall von Fig. 2 beschrieben, auf einem Anzeigegerät (D) angezeigt. Der Benutzer hat mit einem virtuellen Stift (P) eine geschlossene Kurve (C) um die Strukturformel gezogen. Der Bereich innerhalb der Kurve (C) kann für die Erzeugung eines Eingabebildes verwendet werden (siehe hierzu die Beschreibung in Bezug auf Fig. 5).

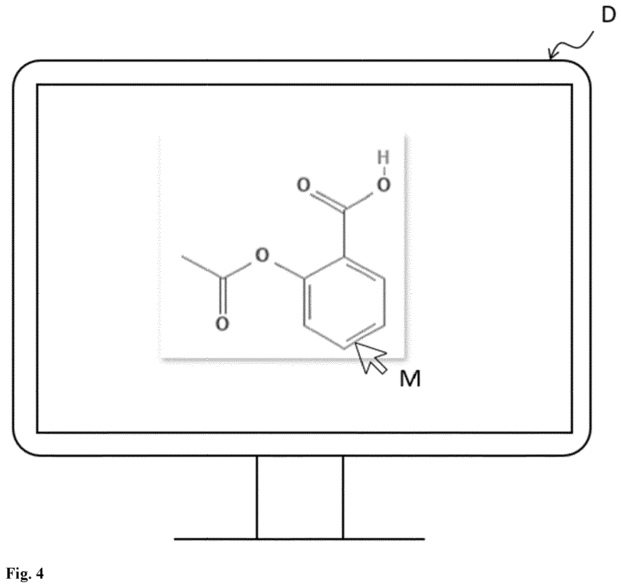

Fig. 4 zeigt beispielhaft und schematisch eine weitere Möglichkeit, wie ein Benutzer eine Strukturformel auswählen kann. Die Strukturformel wird, wie in den Fällen von Fig. 2 und Fig. 3 beschrieben, auf einem Anzeigegerät (D) angezeigt. Bewegt der Benutzer den Mauszeiger (M) über die Strukturformel und/oder berührt der Benutzer mit dem Mauszeiger (M) einen Teil der Strukturformel wird die Strukturformel automatisch ausgewählt. Die Auswahl kann beispielsweise durch Identifizieren von Gruppen zusammenhängender Pixel sowie von Pixeln, bei denen der kürzeste Abstand zu einem anderen Pixel der Strukturformel eine vordefinierte Distanz nicht überschreitet, erfolgen. Dem Benutzer kann angezeigt werden, dass die Strukturformel ausgewählt worden ist, beispielsweise dadurch, dass sie in einer anderen Farbe dargestellt wird, gegenüber dem Hintergrund hervorgehoben und/oder anderweitig markiert/hervorgehoben wird.

Fig. 5 zeigt beispielhaft und schematisch, wie ein Eingabebild auf Basis einer ausgewählten Strukturformel erzeugt werden kann. Im vorliegenden Beispiel wurde eine Strukturformel mit Hilfe des Zeichnens einer geschlossenen Kurve (C) um die Strukturformel ausgewählt. Die Pixel innerhalb der geschlossenen Kurve (C) nehmen im vorliegenden Beispiel einen von zwei Farbtönen an: Pixel, die die Strukturformel repräsentieren sind schwarz; Pixel, die die Strukturformel nicht repräsentieren (sondern den Hintergrund) sind weiß. Die Menge der Pixel innerhalb der geschlossenen Kurve (C) können auf einem rechteckigen neutralen Hintergrund platziert werden. Unter neutral wird ein Hintergrund verstanden, der i) einen anderen Farbwert aufweist als die Pixel, die die Strukturformel repräsentieren, und ii) zu den Pixeln, die die Strukturformel repräsentieren einen möglichst hohen Kontrast aufweist. Vorzugsweise handelt es sich um einen Hintergrund mit demselben Farbwert wie der Hintergrund innerhalb der geschlossenen Kurve (C). Vorzugsweise handelt es sich um einen weißen Hintergrund. Im vorliegenden Beispiel sind zwei Eingangsbilder (I1) und (I2) dargestellt. Sie unterscheiden sich in der Bildgröße; Eingangsbild (I1) ist größer als Eingangsbild (I2). Im vorliegenden Beispiel wurde die Strukturformel in dem Eingangsbild (I1) vergrößert, um den Raum innerhalb des Eingangsbildes mit der Strukturformel auszufüllen. Im Fall des Eingangsbildes (I2) musste die Strukturformel verkleinert werden, um sie in die vom Eingangsbild (I2) definierte Bildgröße einzupassen.

Es ist denkbar, dass von den Eingangsbilder (I1) und/oder (I2) Negative erzeugt werden, bevor sie dem das Modell des maschinellen Lernens zugeführt werden. Auch andere Methoden der Bildverarbeitung sind denkbar, wie beispielsweise Binarisierung, Rauschunterdrückung u.a.

Fig. 6 zeigt beispielhaft und schematisch, wie ein Eingabebild (I) einem trainierten Modell des maschinellen Lernens (MLM$^T$) zugeführt wird. Das Modell des maschinellen Lernens (MLM$^T$) erzeugt auf Basis des Eingabebildes (I) eine eindeutige Kennung (UI).

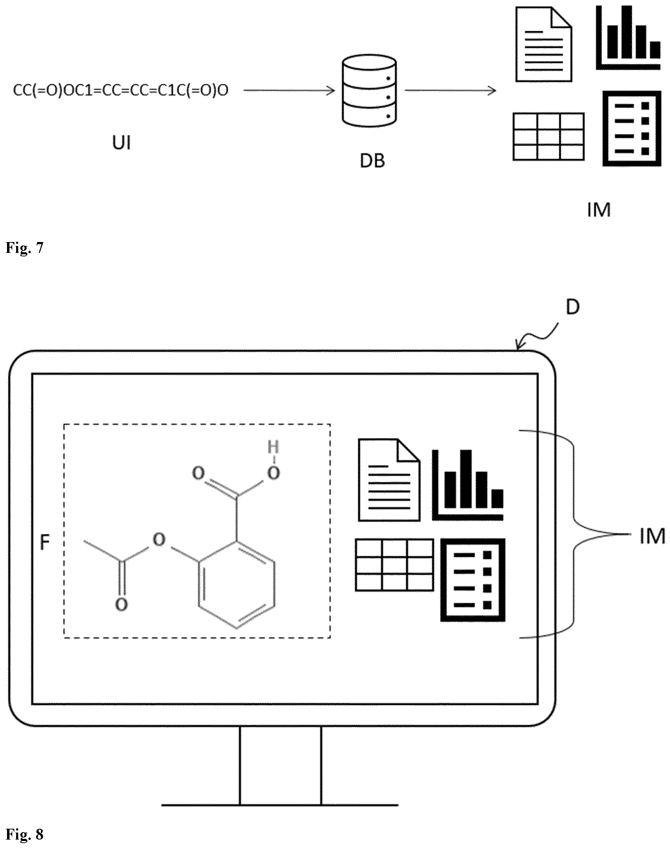

Fig. 7 zeigt beispielhaft und schematisch, wie auf Basis einer eindeutigen Kennung (UI) Informationen (IM) zu einer chemischen Verbindung aus einer Datenbank (DB) abgerufen werden.

Fig. 8 zeigt beispielhaft und schematisch, wie Informationen (IM) zu einer chemischen Verbindung auf einem Anzeigegerät (D) angezeigt werden. Die Informationen (IM) werden im vorliegenden Beispiel direkt neben der Strukturformel angezeigt, die von einem Benutzer mit Hilfe eines Rahmens (F) ausgewählt wurde. Das erfindungsgemäße Computersystem kann konfiguriert sein, dass es die Informationen zu einer chemischen Verbindung automatisch nach der Auswahl durch den Benutzer anzeigt.

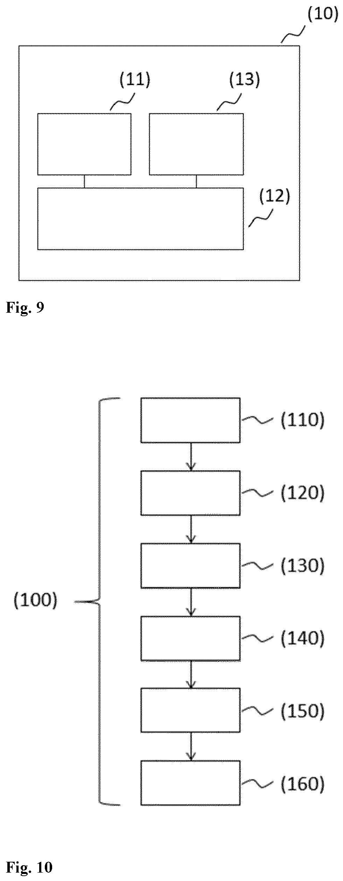

Fig. 9 zeigt schematisch und beispielhaft eine Ausführungsform des erfindungsgemäßen Computersystems. Das Computersystem (10) umfasst eine Eingabeeinheit (11), eine Steuer- und Recheneinheit (12) und ein Anzeigegerät (13).

Das erfindungsgemäße Computersystem (10) ist konfiguriert, von einem Benutzer eine Auswahl über die Eingabeeinheit (11) zu empfangen, wobei die Auswahl eine Strukturformel einer chemischen Verbindung betrifft, die bildhaft auf dem Anzeigegerät (13) angezeigt wird. Das erfindungsgemäße Computersystem (10) ist konfiguriert, die ausgewählte Strukturformel in eine eindeutige Kennung zu transformieren, Informationen zu der chemischen Verbindung anhand der eindeutigen Kennung aus einer oder mehreren Datenbanken abzurufen und die Informationen auf dem Anzeigegerät (13) anzuzeigen. Die eine oder die mehreren Datenbanken können mit dem Computersystem (10) zum Beispiel über ein Netzwerk verbunden sein. Die eine oder die mehreren Datenbanken können aber auch ein Bestandteil des Computersystems (10) sein.

Die Steuer- und Recheneinheit (12) dient der Steuerung der Eingabeeinheit (11) und des Anzeigegeräts (13), der Koordinierung der Daten- und Signalflüsse zwischen den verschiedenen Einheiten, der Prozessierung von digitalen Bildern, der Erzeugung von Eingabebildern, der Erzeugung von eindeutigen Kennungen mittels eines trainierten Modells des maschinellen Lernens und dem Abrufen von Informationen aus einer oder mehreren Datenbanken. Das Modell des maschinellen Lernens kann beispielsweise in einem Arbeitsspeicher des Computersystems (10) geladen sein.

Die Eingabeeinheit (11) dient zum Empfang einer Auswahl eines Benutzers.

Über das Anzeigegerät (13) können Informationen, die aus einer oder mehreren Datenbanken abgerufen wurden, angezeigt werden (zum Beispiel auf einem Monitor).

Fig. 10 zeigt beispielhaft und schematisch eine Ausführungsform des erfindungsgemäßen Verfahrens in Form eines Ablaufdiagramms.

Das Verfahren (100) umfasst die Schritte:

(110) Empfangen einer Auswahl eines Benutzers, wobei die Auswahl eine auf einem Anzeigegerät angezeigte Strukturformel einer chemischen Verbindung betrifft,

(120) Erzeugen eines Eingabebildes der Strukturformel,

(130) Zuführen des Eingabebildes einem Modell des maschinellen Lernens, wobei das Modell des maschinellen Lernens konfiguriert und trainiert ist, eine eindeutige Kennung der chemischen Verbindung auf Basis des Eingabebildes bereitzustellen,

(140) Empfangen der eindeutigen Kennung der chemischen Verbindung von dem Modell des maschinellen Lernens,

(150) Abrufen von Informationen zu der chemischen Struktur auf Basis der eindeutigen Kennung aus einer oder mehreren Datenbanken,

(160) Anzeigen der Informationen auf dem Anzeigegerät.

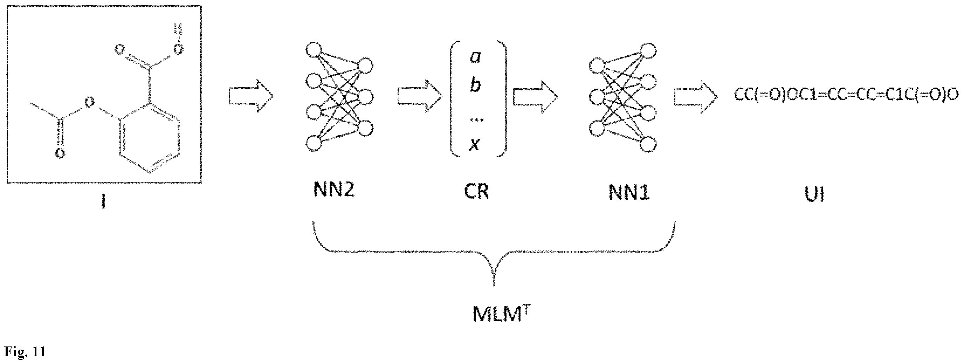

Fig. 11 zeigt beispielhaft und schematisch eine bevorzugte Ausführungsform des Modells des maschinellen Lernens (MLM^T). Das trainierte Modell (MLM^T) umfasst zwei künstliche neuronale Netzwerke (NN1) und (NN2). Das zweite künstliche neuronale Netzwerk (NN2) ist konfiguriert und trainiert ein Eingabebild (I) zu empfangen und eine komprimierte Repräsentation fester Größe (CR) auf Basis des Eingabebildes (I) zu erzeugen. Das erste künstliche neuronale Netzwerk (NN1) ist konfiguriert und trainiert, auf Basis der komprimierten Repräsentation (CR) eine eindeutige Kennung (UI), im vorliegenden Fall einen kanonischen SMILES-Code zu erzeugen.

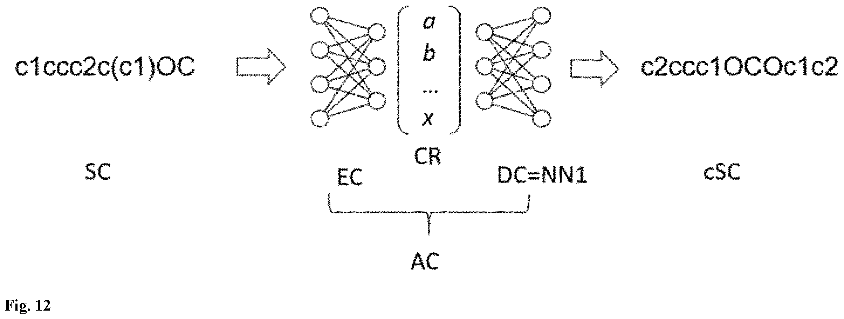

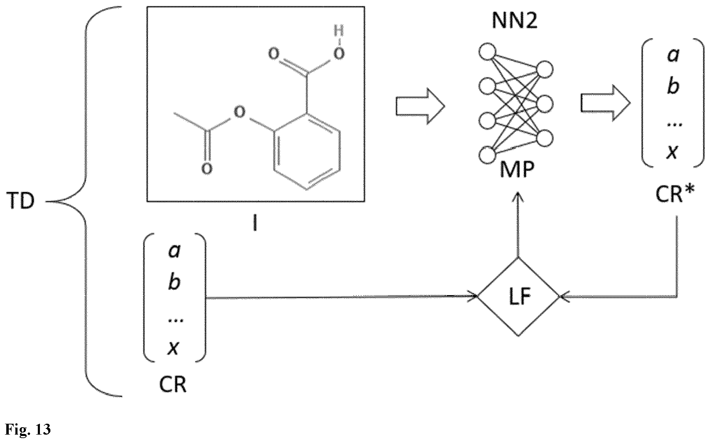

Die künstlichen neuronalen Netzwerke (NN1) und (NN2) können unabhängig voneinander trainiert werden, wie in Fig. 12 und Fig. 13 dargestellt.

Fig. 12 zeigt beispielhaft und schematisch, wie das erste künstliche neuronale Netzwerk (NN1) trainiert werden kann. Das erste künstliche neuronale Netz (NN1) kann als Decoder (DC) eines Autoencoders (AC) konfiguriert sein. Neben dem Decoder (DC) weist der Autoencoder (AC) einen Encoder (EC) auf. Der Encoder (EC) wird trainiert, auf Basis eines SMILES-Codes variabler Größe (SC) eine komprimierte Repräsentation fester Größe (CR) zu erzeugen. Der Decoder (DC) wird trainiert, aus der komprimierten Repräsentation (CR) einen kanonischen SMILES-Code variabler Größe (cSC) zu erzeugen. Encoder (EC) und Decoder (DC) werden gleichzeitig trainiert. Nach dem Trainieren kann der Encoder (EC) verworfen werden.

Fig. 13 zeigt beispielhaft und schematisch, wie das zweite künstliche neuronale Netzwerk (NN2) trainiert werden kann. Das zweite künstliche neuronale Netzwerk (NN2) kann in einem überwachten Lernverfahren trainiert werden. Das Trainieren erfolgt auf Basis von Trainingsdaten (TD). Die Trainingsdaten (TD) umfassen für jede Verbindung einer Vielzahl an chemischen Verbindungen ein Eingabebild (I) einer Strukturformel der chemischen Verbindung und eine komprimierte Repräsentation fester Größe (CR). Im vorliegenden Beispiel sind nur die Trainingsdaten für eine chemische Verbindung dargestellt. Das zweite künstliche neuronale Netzwerk (NN2) wird trainiert, auf Basis eines Eingabebildes (I) die entsprechende komprimierte Repräsentation (CR) zu erzeugen. Das zweite künstliche neuronale Netzwerk (NN2) erzeugt eine Ausgabe (CR*), die mit der komprimierten Repräsentation (CR) verglichen wird. Eine Verlustfunktion (LF) wird verwendet, um die Abweichungen zwischen der Ausgabe (CR*) und den Zieldaten (der komprimierten Repräsentation (CR)) zu quantifizieren. Modellparameter (MP) werden verändert, um die Abweichungen auf ein definiertes Minimum zu reduzieren.

**Patentansprüche**

1. Computer-implementiertes Verfahren umfassend die Schritte:

   - Empfangen einer Auswahl eines Benutzers, wobei die Auswahl eine auf einem Anzeigegerät angezeigte Strukturformel einer chemischen Verbindung betrifft,
   - Erzeugen eines Eingabebildes der Strukturformel,
- Zuführen des Eingabebildes einem Modell des maschinellen Lernens, wobei das Modell des maschinellen Lernens konfiguriert und trainiert ist, eine eindeutige Kennung der chemischen Verbindung auf Basis des Eingabebildes bereitzustellen,
- Empfangen der eindeutigen Kennung der chemischen Verbindung von dem Modell des maschinellen Lernens,
- Abrufen von Informationen zu der chemischen Struktur auf Basis der eindeutigen Kennung aus einer oder mehreren Datenbanken,
- Anzeigen der Informationen auf dem Anzeigegerät.

2. Verfahren gemäß Anspruch 1, wobei es sich bei der eindeutigen Kennung um einen kanonischen SMILES-Code handelt.

3. Verfahren gemäß einem der Ansprüche 1 oder 2,

wobei das Modell des maschinellen Lernens zwei unabhängig voneinander trainierte künstliche neuronale Netzwerke umfasst und/oder aus zwei unabhängig voneinander trainierten künstlichen neuronalen Netzwerken zusammengesetzt ist,
wobei das erste neuronale Netzwerk ein Decoder eines Autoencoders ist, wobei der Autoencoder trainiert wurde, einen SMILES-Code variabler Größe in eine komprimierte Repräsentation fester Größe zu transformieren und aus der Repräsentation einen kanonischen SMILES-Code variabler Größe zu erzeugen,
wobei das zweite neuronale Netzwerk trainiert wurde, auf Basis eines Eingabebildes eine komprimierte Repräsentation fester Größe zu erzeugen.

4. Verfahren gemäß einem der Ansprüche 1 bis 3, umfassend die Schritte:

- Empfangen einer Auswahl eines Benutzers, wobei die Auswahl eine auf einem Anzeigegerät angezeigte Strukturformel einer chemischen Verbindung betrifft,
- Erzeugen eines Eingabebildes der Strukturformel,
- Zuführen des Eingabebildes einem zweiten künstlichen neuronalen Netzwerk, wobei das zweite künstliche neuronale Netzwerk in einem überwachten Lernverfahren trainiert worden ist, für jedes Referenz-Eingabebild einer Vielzahl von Referenz-Eingabebildern von Strukturformeln von chemischen Referenzverbindungen eine komprimierte Repräsentation fester Größe zu erzeugen,

- Empfangen einer komprimierten Repräsentation fester Größe von dem zweiten künstlichen neuronalen Netzwerk,
- Zuführen der komprimierten Repräsentation einem ersten künstlichen neuronalen Netzwerk, wobei das erste künstliche neuronale Netzwerk ein Decoder eines Autoencoders ist, wobei der Autoencoder in einem unüberwachten Lernverfahren anhand einer Vielzahl von chemischen Referenzverbindungen trainiert worden ist, Strukturcodes variabler Größe in komprimierte Repräsentationen fester Größe zu überführen und aus den komprimierten Repräsentationen kanonische Strukturcodes variabler Größe zu erzeugen,
- Empfangen eines kanonischen Strukturcodes variabler Größe von dem ersten künstlichen neuronalen Netzwerk,
- Abrufen von Informationen zu der chemischen Verbindung auf Basis des kanonischen Strukturcodes aus einer oder mehreren Datenbanken,
- Ausgeben der Informationen.

5. Verfahren gemäß einem der Ansprüche 1 bis 4, wobei die Auswahl der Strukturformel durch den Benutzer durch Ziehen eines rechteckigen Rahmens um die Strukturformel oder durch Zeichnen einer geschlossenen Kurve um die Strukturformel erfolgt.

6. Verfahren gemäß einem der Ansprüche 1 bis 4, wobei der Schritt Empfangen einer Auswahl eines Benutzers die folgenden Unterschritte umfasst:

- Erkennen einer Bewegung des Mauszeigers,
- Prüfen, ob ein Teil des Mauszeigers ein Pixel berührt, das zu der Strukturformel gehört,
- Identifizieren von Pixeln, die mit dem berührten Pixel zusammenhängen und/oder von Pixeln, bei denen der kürzeste Abstand zu einem Bestandteil der Strukturformel eine vordefinierte maximale Distanz nicht überschreitet.

7. Verfahren gemäß einem der Ansprüche 1 bis 6, wobei der Schritt Erzeugen eines Eingabebildes der Strukturformel die folgenden Unterschritte umfasst:

- Kopieren von Pixeln innerhalb eines Bereichs, der von dem Benutzer ausgewählt worden ist,
- Platzieren der Pixel auf einer rechteckigen Pixel-Matrix definierter Größe.

8. Computersystem umfassend

• eine Eingabeeinheit,
• eine Steuer- und Recheneinheit und
• ein Anzeigegerät,
- wobei die Steuer- und Recheneinheit konfiguriert ist, die Eingabeeinheit zu veranlassen, eine Auswahl von einem Benutzer zu empfangen, wobei die Auswahl eine auf dem Anzeigegerät angezeigte Strukturformel einer chemischen Verbindung betrifft,

- wobei die Steuer- und Recheneinheit konfiguriert ist, ein Eingabebild der Strukturformel zu erzeugen,

- wobei die Steuer- und Recheneinheit konfiguriert ist, das Eingabebild einem Modell des maschinellen Lernens zuzuführen, wobei das Modell des maschinellen Lernens konfiguriert und trainiert ist, eine eindeutige Kennung der chemischen Verbindung auf Basis des Eingabebildes bereitzustellen,

- wobei die Steuer- und Recheneinheit konfiguriert ist, die eindeutige Kennung der chemischen Verbindung von dem Modell des maschinellen Lernens zu empfangen,

- wobei die Steuer- und Recheneinheit konfiguriert ist, Informationen aus einer oder mehreren Datenbanken anhand der eindeutigen Kennung abzurufen,

- wobei die Steuer- und Recheneinheit konfiguriert ist, das Anzeigegerät zu veranlassen, die Informationen anzuzeigen.

9. Computerprogrammprodukt umfassend ein Computerprogramm, das in einen Arbeitsspeicher eines Computersystems geladen werden kann und dort das Computersystem dazu veranlasst, folgende Schritte ausführen:

> - Empfangen einer Auswahl eines Benutzers, wobei die Auswahl eine auf einem Anzeigegerät angezeigte Strukturformel einer chemischen Verbindung betrifft,
> - Erzeugen eines Eingabebildes der Strukturformel,
> - Zuführen des Eingabebildes einem Modell des maschinellen Lernens, wobei das Modell des maschinellen Lernens konfiguriert und trainiert ist, eine eindeutige Kennung der chemischen Verbindung auf Basis des Eingabebildes bereitzustellen,
> - Empfangen der eindeutigen Kennung der chemischen Verbindung von dem Modell des maschinellen Lernens,
> - Abrufen von Informationen zu der chemischen Struktur auf Basis der eindeutigen Kennung aus einer oder mehreren Datenbanken,
> - Anzeigen der Informationen auf dem Anzeigegerät.

**Fig. 1**

**Fig. 2**

Fig. 3

Fig. 4

**Fig. 5**

**Fig. 6**

CC(=O)OC1=CC=CC=C1C(=O)O

UI

DB

IM

Fig. 7

D

F

IM

Fig. 8

**Fig. 9**

**Fig. 10**

CC(=O)OC1=CC=CC=C1C(=O)O

UI

NN1

MLM$^T$

CR

a
b
...
x

NN2

I

Fig. 11

Fig. 12

Fig. 13

Europäisches Patentamt
European Patent Office
Office européen des brevets

# EUROPÄISCHER RECHERCHENBERICHT

Nummer der Anmeldung

EP 21 21 6717

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | CLEVERT DJORK-ARNÉ ET AL: "Img2Mol – accurate SMILES recognition from molecular graphical depictions", CHEMICAL SCIENCE, Bd. 12, Nr. 42, 29. September 2021 (2021-09-29), Seiten 14174-14181, XP055928510, United Kingdom ISSN: 2041-6520, DOI: 10.1039/D1SC01839F Gefunden im Internet: URL:https://pubs.rsc.org/en/content/articlepdf/2021/sc/d1sc01839f> * Zusammenfassung * * Seite 14175, Spalte 2, Absatz 3; Abbildungen 2,3 * * Seite 14176, Spalte 2, Absatz 8 – Seite 14177, Spalte 1, Absatz 1 * ----- | 1-9 | INV. G16C20/80 G16C20/40 |
| Y | KIM SUNGHWAN ET AL: "PubChem Substance and Compound databases", NUCLEIC ACIDS RESEARCH, Bd. 44, Nr. D1, 4. Januar 2016 (2016-01-04), Seiten D1202-D1213, XP055928544, GB ISSN: 0305-1048, DOI: 10.1093/nar/gkv951 Gefunden im Internet: URL:https://www.ncbi.nlm.nih.gov/pmc/articles/PMC4702940/pdf/gkv951.pdf> * Zusammenfassung; Tabelle 1 * * Seite D1206, Spalte 2, Absatz 2 * * Seite D1207, Spalte 1, Absatz 5 * ----- | 1-9 | RECHERCHIERTE SACHGEBIETE (IPC) G16C |
| Y | EP 3 876 236 A1 (MERCK PATENT GMBH [DE]) 8. September 2021 (2021-09-08) * Absätze [0008], [0014] – [0017] * ----- | 1-9 | |

−/--

Der vorliegende Recherchenbericht wurde für alle Patentansprüche erstellt

| Recherchenort | Abschlußdatum der Recherche | Prüfer |
|---|---|---|
| Den Haag | 7. Juni 2022 | Nurmi, Jussi |

EPO FORM 1503 03.82 (P04C03)

Seite 1 von 2

Europäisches
Patentamt

European
Patent Office

Office européen
des brevets

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | ROBIN WINTER ET AL: "Learning continuous and data-driven molecular descriptors by translating equivalent chemical representations", CHEMICAL SCIENCE, Bd. 10, Nr. 6, 19. November 2018 (2018-11-19), Seiten 1692-1701, XP055712488, United Kingdom ISSN: 2041-6520, DOI: 10.1039/C8SC04175J * Zusammenfassung; Abbildung 1 * * Seite 1693, Spalte 1, Absatz 3 * ----- | 1 | |

RECHERCHIERTE
SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 7. Juni 2022 | Nurmi, Jussi |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

 

& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 21 6717

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-06-2022

| Im Recherchenbericht angeführtes Patentdokument | Datum der Veröffentlichung | Mitglied(er) der Patentfamilie | Datum der Veröffentlichung |
|---|---|---|---|
| EP 3876236 A1 | 08-09-2021 | KEINE | |

Für nähere Einzelheiten zu diesem Anhang : siehe Amtsblatt des Europäischen Patentamts, Nr.12/82

EPO FORM P0461

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 8121434 B2
- WO 2018046412 A1

**In der Beschreibung aufgeführte Nicht-Patentliteratur**

- **J. K. FELIXBERGER.** Chemie für Einsteiger. Springer-Verlag, 2017
- **S. FEIL et al.** Faszinierende Chemie. Springer-Verlag, 2017, 32-33
- **E. BENFENATI et al.** Characterization of chemical structures, Chapter 3 of Quantitative Structure-Activity Relationships (QSAR) for Pesticide Regulatory Purposes. Elsevier-Verlag, 2007, 83-109
- **T. FRAJKA et al.** Downsampling dependent upsampling of images. *Signal Processing: Image Communication,* 2004, vol. 19 (3), 257-265
- **M.-S. LEE et al.** *An Efficient Upsampling Technique for Images and Videos*
- Advances in Multimedia Information Processing - PCM. Lecture Notes in Computer Science. Springer, 2009, vol. 5879
- **D. WEININGER et al.** SMILES. 2. algorithm for generation of unique SMILES notation. *J Chem Inf Comp Sci,* 1989, vol. 29 (2), 97e101
- **WINTER et al.** *Chem. Sci.,* 2019, vol. 10, 1692-1701
- **Q. V. LE.** A Tutorial on Deep Learning Part 2: Autoencoders. *Convolutional Neural Networks and Recurrent Neural Networks,* 2015, https://cs.stanford.edu/~quocle/tutorial2.pdf
- **W. MENG.** Relational Autoencoder for Feature Extraction. *arXiv: 1802.03145vl*
- **F. NIE et al.** An investigation for loss functions widely used in machine learning. *Communications in Information and Systems,* 2018, vol. 18, 37-52
- **VON CLEVERT et al.** beschriebene Architektur für das zweite neuronale Netzwerk verwendet (Img2Mol - accurate SMILES recognition from molecular graphical depictions. *Chem. Sci.,* 2021, vol. 12, 14174-14181, https://doi.org/10.1039/D1SC01839F